# EUROPEAN PATENT APPLICATION

(11) **EP 3 087 979 A1**
(43) Date of publication of application: **02.11.2016**
(21) Application number: 14875660.4
(22) Date of filing: 19.12.2014
(51) Int. Cl.: A61K 9/22, A61K 9/28, A61K 47/38, A61K 31/522

(54) **SUSTAINED-RELEASE PHARMACEUTICAL COMPOSITION CONTAINING ACEBROPHYLLINE AND HYDROPHOBIC SUSTAINED-RELEASE AGENT**

(30) Priority: 26.12.2013 KR 20130164670
(71) Applicant: Hyundai Pharm Co., Ltd., Cheonan-si, Chungcheongnam-do 330-911 (KR)
(72) Inventor: LEE, Pung Sok, Yongin-si Gyeonggi-do 446-718 (KR); LEE, Chung Lyol, Icheon-si Gyeonggi-do 467-822 (KR); KIM, Seong Yeon, Goyang-si Gyeonggi-do 412-220 (KR); LEE, Dong Il, Seongnam-si Gyeonggi-do 463-876 (KR); YOON, Min Ji, Seoul 152-050 (KR)
(74) Representative: Krauss, Jan
(86) International application number: PCT/KR2014/012602
(87) International publication number: WO 2015/099364

(57) **Abstract**

The present invention relates to a pharmaceutical composition containing acebrophylline thereby having both immediate and extended release characteristics, and more specifically, to a sustained-release pharmaceutical composition containing acebrophylline and a hydrophobic sustained-release agent.

## Description

### Technical Field

The present invention relates to a sustained-release pharmaceutical composition containing acebrophylline and a hydrophobic sustained-release agent.

### Background Art

Acebrophylline is a compound synthesized by salifying ambroxol with 7-theophylline, and a salt composed of an acid and a base. Acebrophylline is metabolized by being separated into ambroxol and 7-theophylline when orally administered. Acebrophylline is selectively applied to the bronchial or lung tissue to inhibit the activity of phospholipase in bronchial alveolus, and to improve the alveolar surface activity, thereby exhibiting the sputum-removal action. In addition, acebrophylline suppresses the production of leukotrienes (LTs) and prostaglandins (PGs) to exhibit a strong anti-inflammatory action, and thus recovers or expands the convulsed bronchial tubes to a normal state by deteriorating bronchial hypersensitiveness.

Acebrophylline preparations previously licensed in Korea (for example, product name, Surfolase® capsule (Hyundai Pharmaceutical), of which 100 mg is administered twice daily, as compared with a drug administered once daily or a drug administered thrice daily (before and after meals)), have unclear administration time points, causing a reduction in patient drug compliance and an irregular administration time (interval), resulting in undesired therapeutic effects. In order to improve the drug compliance, it is preferable to develop sustained-release preparations that can exhibit continuous drug efficacy. However, such sustained-release preparations exhibit only continuous drug efficacy without fast exhibition of drug efficacy, and thus cannot be expected to have fast efficacy after administration in, for example, respiratory diseases that are in need of fast exhibition of drug efficacy, so the sustained-release preparations increase the inconvenience of patients and are not expected to produce desired therapeutic effects as compared with the preparations on the market. Accordingly, in order to complement these defects, acebrophylline preparations need to be developed that have both a fast-acting property to rapidly reach an effective blood concentration after administration to exhibit drug efficacy, and also have a long-acting property to sustain drug efficacy for a long time.

Thus, the present inventors studied to develop acebrophylline preparations that have both immediate-release characteristics of rapidly reaching an effective blood concentration after administration to exhibit drug efficacy and sustained-release characteristics of sustaining drug efficacy for a long time. As a result, the present inventors developed an acebrophylline preparation that, while exhibiting fast drug efficacy and sustaining therapeutic effects, has an improved the convenience of administration by pharmaceutically modifying the acebrophylline administration schedule from 100 mg twice daily into 200 mg once daily, and thus completed the present invention.

### Detailed Description of the Invention

### Technical Problem

An aspect of the present invention is to provide a pharmaceutical composition containing acebrophylline and simultaneously having a fast-acting property, a long-acting property, and the convenience of administration, simultaneously.

Another aspect of the present invention is to provide a method for imparting, to acebrophylline, initial immediate-release characteristics and late sustained-release characteristics.

### Technical Solution

In accordance with an aspect of the present invention, there is provided a sustained-release pharmaceutical composition containing acebrophylline and a hydrophobic sustained-release agent.

Hereinafter, the present invention will be described in detail.

The present invention tested to prepare a pharmaceutical composition containing acebrophylline, and as a result, verified that the preparation manufactured according to the present invention has an excellent initial release rate compared with a preparation including an immediate-release layer, thereby expecting a fast therapeutic effect (test example 1), and only once daily administration of the preparation of the present invention can exhibit a similar blood acebrophylline concentration as a twice daily administration of an existing preparation (test examples 2 to 4), thereby improving the convenience of administration and expecting an improved therapeutic effect.

The pharmaceutical composition of the present invention contains acebrophylline as an active ingredient, and exhibits a fast dissolution rate although it contains the hydrophobic sustained-release agent, and thus has both a fast-acting property and a long-acting property.

Here, acebrophylline is a compound (ambroxol[trans-4-2-amino-3,5-dibromobenzy]amino cyclohexanol with theophylline-7-acetic acid[1,3-dimethylxanthine-7-acetic acid]) that is synthesized by salifying ambroxol with 7-theophylline and a salt composed of an acid and a base.

Acebrophylline is metabolized by being separated into ambroxol and 7-theophylline when orally administered. Acebrophylline is selectively applied to the lung tissue to exhibit the sputum-removal action, and inhibits the production of leukotrienes and prostaglandins to exhibit an anti-inflammatory action. Therefore, acebrophylline can be used in the prevention or treatment of acute respiratory disease, chronic respiratory disease, acute bronchitis, chronic bronchitis, bronchial asthma, sinusitis, or rhinitis sicca.

Therefore, preferably, the present invention achieves fast drug efficacy exhibition and continuous drug efficacy retention of acebrophylline, and may be used in the prevention or treatment of at least one disease selected from the group consisting of acute respiratory disease, chronic respiratory disease, acute bronchitis, chronic bronchitis, bronchial asthma, sinusitis, and rhinitis sicca.

As a preferable example, the content of acebrophylline contained in the composition of the present invention may be easily determined by a person skilled in the art depending on the age, sex, disease severity, weight, and the like of a patient, but may be, preferably, a total of 20-250 mg, and more preferably, a total of 200 mg. Less than 20 mg of acebrophylline may not sufficiently exhibit desired drug efficacy, and more than 250 mg of acebrophylline may cause side effects due to an excessive pharmacological action and may spoil administration due to an increase in the weight of the composition *per se,* and thus is not useful as a pharmaceutical composition.

Here, the hydrophobic sustained-release agent refers to a hydrophobic material that is used to release drugs for a long time. The term "hydrophobic" means the immiscibility with water molecules. Broadly, hydrophobic materials have no polarity, and thus are well dissolved in nonpolar solvents.

Examples of the hydrophobic sustained-release agent may include, but are not limited to, at least one selected from the group consisting of cellulose-based hydrophobic polymers (e.g., cellulose acetate, cellulose acetate phthalate, and ethylcellulose), vegetable waxes (e.g., carnauba wax), glyceryl-based hydrophobic materials (e.g., glycerin monostearate, glyceryl monooleate, glyceryl palmitostearate, polyoxyglycerides, and glyceryl behenate), vegetable oils (e.g., hydrogenated castor oil, hydrogenated vegetable oil type I), hydrophobic fatty acids (e.g., stearic acid and isopropyl palmitate), acetyltributyl citrate, tributyl citrate, white wax, yellow wax, zein, acetyl alcohol, acetyl esters wax, dibutyl sebacate, polymethacrylates, shellac, stearyl alcohol, and a mixture thereof.

According to an embodiment of the present invention, the polymethacrylates are polyalkylmethacrylates, and an example thereof is, specifically, a copolymer of ethyl acrylate, methyl methacrylate, and ester methacrylate having a quaternary ammonium group, and more specifically, a copolymer of ethyl acrylate, methyl methacrylate, and ester methacrylate having a quaternary ammonium group.

Specific examples of the polymethacrylate used herein may be selected from the group consisting of, but are not limited thereto, Eudragit RS100, RS PO, RS 30D, RL100, RL PO, RL 30D, NE30D, NE40D, FS30D, and NM30D, which are available as polymethyl methacrylate copolymers.

More specifically, suitable examples of the hydrophobic sustained-release agent are cellulose-based hydrophobic polymers (exemplified in example 1, more specifically, C₁-C₅ alkyl cellulose, and still more specifically, ethyl cellulose), vegetable waxes (exemplified in example 2, more specifically, carnauba wax), glyceryl-based hydrophobic materials (exemplified in example 3, more specifically, glycerides having C₁₄-C₂₂ fatty acids bound to glycerol, and still more specifically, glyceryl behenate), or polymethacrylates (exemplified in example 4, more specifically, a copolymer of ethyl acrylate, methyl methacrylate, and ester methacrylate having a quaternary ammonium group, and still more specifically, a copolymer of ethyl acrylate, methyl methacrylate, and ester methacrylate having a quaternary ammonium group).

The suitable hydrophobic sustained-release agent is, more specifically, a cellulose-based hydrophobic polymer or vegetable wax, still more specifically, a cellulose-based hydrophobic polymer, and most specifically, ethylcellulose.

In addition, the preferable content of the hydrophobic agent is, but is not limited to, 10-50 wt% (more preferably, 15-40 wt%) on the basis of the entire weight of the unit dosage form. The sustained-release agent allows the pharmaceutical composition of the present invention to release 80% or more of the active ingredient within 6 hours, thereby exhibiting the drug efficacy through only once-daily administration. The administration of less than the above content is insufficient to exhibit the above effect, and the administration of more than the above content is not economical, and increases the entire weight of the unit dosage form, causing an inconvenience of administration.

According to still another aspect of the present invention, the composition of the present invention may further contain a coating agent. An example of the coating agent may be at least one selected from the group consisting of hydroxypropyl methylcellulose, polyvinyl alcohol, polypropylene glycol, acrylic acid polymers, polymethacrylate copolymers, polyethylene glycol, polyvinyl acetate, and a mixture thereof, but is not limited thereto.

In addition, the composition may further contain a pharmaceutically acceptable additive, and a general additive that can improve the manufacturing, external appearance, and compression of a drug may be used without deteriorating the biological activity and characteristics of the drug. The specific content thereof may be determined by the solubility and chemical characteristics of the active ingredient, selected route of administration, and standard pharmaceutical practice. Examples of the pharmaceutically acceptable additive may include an excipient, a diluent, a binder, a lubricant, a preservative, a stabilizer, an anti-adhesive, a fluidizer, or a colorant.

More specifically, starch, microcrystalline cellulose, lactose, glucose, mannitol, dicalcium phosphate, etc., may be used as the diluent; hydroxypropyl cellulose, polyethylene glycol, polyvinyl pyrrolidone, polyvinyl alcohol, a polyvinyl pyrrolidone derivative such as copovidone, natural gum, synthetic gum, gelatin, etc., may be used as the binder; and light anhydrous silicic acid, talc, magnesium stearate, calcium stearate, zinc stearate, sodium stearyl fumarate, etc., may be used as the lubricant. Besides, various additives, such as an antioxidant, a colorant, a flavoring agent, a preservative, and a taste masking agent, may be selected and used within general dose ranges by a person skilled in the art.

According to an embodiment of the present invention, the pharmaceutical composition of the present invention has an acebrophylline dissolution rate of 20-52 wt% (more specifically, 20-50 wt%, and still more specifically, 30-50 wt%) at 1 hour after dissolution, and an acebrophylline dissolution rate of 75-100 wt% (more specifically, 80-100 wt%, and still more specifically, 85-100 wt%) at 6 hours after dissolution. Thus, when administered into the body, the pharmaceutical composition may exhibit favorable drug efficacy even under a reduced frequency of administration, for example, once daily. Therefore, the patient convenience of administration can be improved by using the pharmaceutical composition.

As used herein, the term "dissolution rate", unless otherwise mentioned, is obtained by: initiating a dissolution test in 500 mL of a dissolution medium (first fluid in Korean pharmacopoeia disintegration test) of pH 1.2 under 37±0.5□ and 50 rpm using second method 2 (paddle method) of Korean pharmacopoeia; carefully adding 400 mL of a test solution 2(second fluid, 0.235 M anhydrous Na₂HPO₄ solution) to the dissolution medium of pH 1.2 to make a liquid of pH 6.8 at 2 hours after the initiation of the dissolution test; and then continuously performing the dissolution test. The first fluid is prepared by dissolving 2.0 g of sodium chloride in 7.0 mL of hydrochloric acid and water to make 100 mL, and the second fluid is prepared by adding 118 mL of 0.2 mol/L sodium hydroxide solution and water to 250 mL of 0.2 mol/L potassium dihydrogen phosphate solution to make 100 mL.

The pharmaceutical composition of the present invention may preferably be formulated in an administration form suitable to be orally administered, but is not limited thereto, and may preferably be provided in a solid oral dosage form in consideration of the productivity of producers and the convenience of administration and portability of patients.

As a specific example, the solid oral dosage form may be, for example, a single tablet, a multi-layered tablet, such as a double-layered tablet or a triple-layered tablet, a core tablet, a coated tablet, or a capsule, but is not limited thereto. As a preferable example, the solid oral dosage form may be a monolithic matrix type sustained-release tablet containing acebrophylline or a salt thereof as an active ingredient, a hydrophobic sustained-release agent for controlling the dissolution of the active ingredient, an additive, and a coating agent.

According to an embodiment of the present invention, the composition of the present invention is provided in the form of a monolithic matrix type tablet.

According to an embodiment of the present invention, the composition of the present invention has both immediate-release characteristics and sustained-release characteristics. As used herein to recite the composition of the present invention, unless otherwise mentioned, the term "immediate-release characteristics" refers to the rapid release of a drug at an initial stage after administration into the human body (e.g., at 2-4.5 hours, 2-4 hours, 2.5-4 hours, or 3-4 hours after administration). The composition of the present invention has both immediate-release characteristics in which a drug can rapidly reach an effective blood concentration after administration (e.g., oral administration) to exhibit drug efficacy, and sustained-release characteristics in which the drug efficacy can be retained for a long period of time.

According to an embodiment of the present invention, the composition of the present invention shows the maximal blood ambroxol concentration at 2-4.5 hours (more specifically, 2-4 hours, 2.5-4 hours, or 3-4 hours) after once daily oral administration into the human body, thus exhibiting immediate-release characteristics, and then exhibits sustained-release characteristics.

According to an embodiment of the present invention, when 200 mg of acebrophylline is orally administered once daily to the human body, the composition of the present invention has a maximal blood ambroxol concentration of 90-250 ng/mL (more specifically, 100-250 ng/mL, 150-250 ng/mL, 200-250 ng/mL, 100-220 ng/mL, 150-220 ng/mL, or 200-220 ng/mL).

According to an embodiment of the present invention, the composition of the present invention has an AUC_{(0-36 hr)} value of ambroxol in the blood, of 1, 600-2,700 ng·hr /mL when 200 mg of acebrophylline is orally administered once daily to the human body. The term "AUC_{(0-36 hr)} value" refers to an AUC value when the blood ambroxol concentration is measured during 36 hours after oral administration.

The particular type or dose of the agent of the present invention may be selected depending on characteristics of the patient, especially, age, body weight, and lifestyle of the patient and, in some cases, by a doctor, and preferably, the composition of the present invention may be prepared such that 200 mg of acebrophylline is administered once daily in order to improve patient compliance.

Each dosage form usable in the present invention may be obtained by methods generally known in the art. For example, dry granulation, wet granulation, melt granulation, fluidized-bed granulation, direct compression, molding, compressive molding, and the like may be carried out. Preferably, dry granulation, wet granulation, and melt granulation may be carried out. For example, in the case of direct compression method, the composition of the present invention may be prepared in an oral dosage form by mixing acebrophylline and a pharmaceutically acceptable additive, followed by tableting and coating. In addition, in the case of dry granulation method, the composition of the present invention may be prepared by mixing acebrophylline and a pharmaceutically acceptable additive, and then forming granules from the mixture using a compression granulator(roller compactor), followed by sieving, tableting, and coating. In addition, in the case of wet granulation method, the composition of the present invention may be prepared by mixing acebrophylline and a pharmaceutically acceptable additive, forming wet granules from the mixture using a binder liquid, and then drying the wet granules, followed by sieving, tableting, and coating.

The acebrophylline-containing preparation according to the present invention is very useful since the preparation is orally administered to exhibit a prompt therapeutic effect; the active ingredient is continuously sustained at the predetermined concentration in the blood plasma for a considerable time; the dosing frequency is decreased to once daily; and resultantly, with respect to patients, the drug adaptability is increased and the drug compliance is improved.

In accordance with another aspect of the present invention, there is provided a method for imparting initial immediate-release characteristics and late sustained-release characteristics to a pharmaceutical composition containing acebrophylline, the method including a step of adding a hydrophobic sustained-release agent to the pharmaceutical composition containing acebrophylline, followed by mixing

Since the method of the present invention is directly associated with the above-described pharmaceutical composition, descriptions of overlapping contents between the two are omitted to avoid excessive complexity of the specification due to repetitive descriptions thereof.

As used herein, the term "initial immediate-release" refers to an acebrophylline dissolution rate of 20-52 wt% at 1 hour in the dissolution liquid of pH 1.2 when the dissolution rate is analyzed by the above-described paddle method, or a rapid release (e.g., exhibiting the maximal blood concentration) of a drug, for example, at 2-4.5 hours, 2-4 hours, 2.5-4 hours, or 3-4 hours after administration, when a pharmaceutical composition (e.g., tablet) is administered to the human body (e.g., oral administration).

As used herein, the term "late sustained-release" refers to a sustained-release occurring after the dissolution of acebrophylline occurs by the foregoing initial immediate-release. For example, the pharmaceutical composition of the present invention may exhibit an initial immediate-release, a rapidly reduced release rate, a reduced release rate, and then sustained-release. Alternatively, the pharmaceutical composition of the present invention may exhibit an initial immediate-release, a gently reduced release rate, and then sustained-release at such a reduced release rate.

The method of the present invention may be performed in various manners.

For example, for manufacturing tablets, the present invention may be performed by including: (a) mixing a diluent and a binder with acebrophylline as an active ingredient (optionally, a lubricant may be added), followed by granulation; and (b) adding an excipient, and a hydrophobic sustained-release agent capable of imparting initial immediate-release characteristics and late sustained-release characteristic to the resultant material in step (a) (optionally, a lubricant may be added), followed by mixing and tableting. Optionally, the tablet prepared after the tableting may be coated.

According to an embodiment of the present invention, the pharmaceutical composition mixed with the hydrophobic sustained-release agent shows an acebrophylline dissolution rate of 20-52 wt% at 1 hour after dissolution, and an acebrophylline dissolution rate of 70-100 wt% at 6 hours after dissolution.

According to an embodiment of the present invention, the hydrophobic sustained-release agent used in the present invention is at least one selected from the group consisting of acetyltributyl citrate, carnauba wax, cellulose acetate, glycerin monostearate, glyceryl monooleate, glyceryl palmitostearate, hydrogenated castor oil, polyoxyglycerides, stearic acid, tributyl citrate, white wax, yellow wax, zein, cellulose acetate phthalate, acetyl alcohol, acetyl esters wax, dibutyl sebacate, ethylcellulose, glyceryl behenate, hydrogenated vegetable oil type I, isopropyl palmitate, polymethacrylates, shellac, stearyl alcohol, and a mixture thereof.

According to an embodiment of the present invention, the pharmaceutical composition mixed with the hydrophobic sustained-release agent is provided in a solid oral dosage form.

According to an embodiment of the present invention, the pharmaceutical composition mixed with the hydrophobic sustained-release agent is provided in the form of a monolithic matrix type tablet.

According to an embodiment of the present invention, the pharmaceutical composition mixed with the hydrophobic sustained-release agent shows a maximal blood ambroxol concentration at 2-4.5 hours after once daily oral administration to the human body, thereby exhibiting immediate-release characteristics, and then exhibits sustained-release characteristics.

According to an embodiment of the present invention, the pharmaceutical composition mixed with the hydrophobic sustained-release agent shows a maximal blood ambroxol concentration of 90-250 ng/mL when 200 mg of acebrophylline is orally administered once daily to the human body.

According to an embodiment of the present invention, the pharmaceutical composition mixed with the hydrophobic sustained-release agent has an AUC_{(0-36 hr)} value of 1,600-2,700 ng·hr /mL of ambroxol in the blood when 200 mg of acebrophylline is orally administered once daily to the human body.

### Advantageous Effects

The acebrophylline-containing monolithic matrix type sustained-release preparation provided according to the present invention is designed to allow a simple preparing process and exhibit both a fast-acting property and a long-acting property, and thus can secure an excellent therapeutic effect with only once daily administration and increase drug adaptability, thereby expecting significantly improved compliance of administration and excellent therapeutic effect.

### Brief Description of the Drawings

FIG. 1 shows dissolution test results of preparations obtained according to examples 1-4 and 2-4, and comparative example 1.
FIG. 2 shows *in vivo* human pharmacokinetics (PK) test results of a Surfolase® capsule on the market and a preparation obtained according to comparative example 1.
FIG. 3 shows *in vivo* human PK test results of a Surfolase® capsule on the market and a preparation obtained according to example 1-4.
FIG. 4 shows *in vivo* human PK test results of a Sulfolase capsule on the market and a preparation obtained according to example 2-4.

### Mode for Carrying Out the Invention

Hereinafter, the present invention will be described in detail with reference to the following examples. However, the following examples are merely for illustrating the present invention and are not intended to limit the scope of the present invention.

### Example 1: Manufacture of hydrophobic sustained-release preparation containing ethylcellulose

Components 1 to 4 on table 1 were mixed, and made into granules. Components 5 to 7 on table 1 were added to the granules, followed by mixing and tableting. After the tableting, the tablets were coated with component 8 as a coating agent.

**[Table 1]**

| | Ingredient | Weight ratio (%) | | | |
|---|---|---|---|---|---|
| | | Example 1-1 | Example 1-2 | Example 1-3 | Example 1-4 |
| 1 | Acebrophylline (active pharmaceutical ingredient) | 32.9 | 34.0 | 35.8 | 37.2 |
| 2 | Microcrystalline cellulose (diluent) | 28.0 | 28.9 | 30.5 | 31.6 |
| 3 | Talc (lubricant) | 1.6 | 1.7 | 1.8 | 1.9 |
| 4 | Hydroxypropyl cellulose (binder) | 2.6 | 2.7 | 2.9 | 3.0 |
| 5 | Ethylcellulose (sustained-relase agent) | 32.9 | 30.6 | 26.9 | 24.2 |
| 6 | Calcium phosphate dihydrate (excipient) | 1.0 | 1.0 | 1.1 | 1.1 |
| 7 | Magnesium stearae (lubricant) | 1.0 | 1.0 | 1.1 | 1.1 |
| | **Total (plain tablet)** | **100.0** | **100.0** | **100.0** | **100.0** |
| 8 | Opadray (coating agent) | 3.6 | 4.1 | 4.7 | 4.8 |
| | **Total (coated tablet)** | **103.6** | **104.1** | **104.7** | **104.8** |

### Example 2: Manufacture of hydrophobic sustained-release preparation containing wax

Components 1 to 4 on table 2 were mixed, and made into granules. Components 5 to 6 on table 2 were added to the granules, followed by mixing and tableting. After the tableting, the tablet was coated with component 7 as a coating agent.

**[Table 2]**

| | Ingredient | Weight ratio (%) | | | |
|---|---|---|---|---|---|
| | | Example 2-1 | Example 2-2 | Example 2-3 | Example 2-4 |
| 1 | Acebrophylline | 35.7 | 37.7 | 41.6 | 43.4 |
| 2 | Microcrystalline cellulose | 26.7 | 28.2 | 31.2 | 32.5 |
| 3 | Talc | 1.8 | 1.9 | 2.1 | 2.2 |
| 4 | Hydroxypropyl cellulose | 2.9 | 3.0 | 3.3 | 3.5 |
| 5 | Carnauba wax | 32.1 | 28.2 | 20.8 | 17.4 |
| 6 | Magnesium stearate | 0.9 | 0.9 | 1.0 | 1.1 |
| | **Total (plain tablet)** | **100.0** | **100.0** | **100.0** | **100.0** |
| 7 | Opadry | 3.9 | 4.1 | 4.6 | 4.8 |
| | **Total (coated agent)** | **103.9** | **104.1** | **104.6** | **104.8** |

### Example 3: Manufacture of hydrophobic sustained-release preparation containing glyceryl monobehenate

Components 1 to 4 on table 3 were mixed, and made into granules. Components 5 to 6 on table 3 were added to the granules, followed by mixing and tableting. After the tableting, the tablet was coated with component 7 as a coating agent.

**[Table 3]**

| | Ingredient | Weight ratio (%) |
|---|---|---|
| | | Example 3 |
| 1 | Acebrophylline | 44.4 |
| 2 | Microcrystalline cellulose | 26.7 |
| 3 | Talc | 2.2 |
| 4 | Hydroxypropyl cellulose | 3.3 |
| 5 | Glyceryl monobehenate | 22.2 |
| 6 | Magnesium stearate | 1.1 |
| | **Total (plain tablet)** | **100.0** |
| 7 | Opadry | 4.0 |
| | **Total (coated agent)** | **104.0** |

### Example 4: Manufacture of acebrophylline-containing hydrophobic sustained-release preparation containing polymethacrylate

Components 1 to 3 on table 4 were mixed, and made into granules. Components 4 to 6 on table 4 were added to the granules, followed by mixing and tableting. After the tableting, the tablet was coated with component 7 as a coating agent.

**[Table 4]**

| 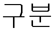 | Ingredient | Weight ratio (%) |
|---|---|---|
| | | Example 4 |
| 1 | Acebrophylline | 44.4 |
| 2 | Microcrystalline cellulose | 18.9 |
| 3 | Talc | 2.2 |
| 4 | Eudragit RS PO* | 18.3 |
| 5 | Eudragit RL PO* | 15.0 |
| 6 | Magnesium stearate | 1.1 |
| | **Total (plain tablet)** | **100.0** |
| 7 | Opadry | 3.0 |
| | **Total (coated agent)** | **103.0** |

| | | |
|---|---|---|
| * copolymer of ethylacrylate, methyl methacrylate, and ester methacrylate having tertiary ammonium group | | |

### Comparative example 1: Manufacture of double-layered tablet composed of immediate-release layer and sustained-release layer

For the sustained-release layer, dry granules were formed by mixing the components on table 5 and compressing the mixture using a roller compactor. For the immediate-release layer, granules were formed by mixing the components on table 5. The granules formed for the sustained-release layer and the immediate-release layer were made into double-layered tablets through tableting. After the tableting, the tablets were coated with a coating agent (Opadry) with a weight of 6% on the basis of the weight of the double-layered tablet.

**[Table 5]**

| | Ingredient | Comparative example 1 | Note |
|---|---|---|---|
| | | Weight ratio (%) | |
| 1 | Acebrophylline | 10.8 | |
| 2 | Lactose hydrate | 5.9 | |
| 3 | Microcrystalline cellulose | 5.3 | Immediate-release layer |
| 4 | Croscarmellose sodium | 2.6 | |
| 5 | Mannitol | 16.9 | |
| 6 | Silicon dioxide | 0.9 | |
| 7 | Magnesium stearate | 0.9 | |
| 8 | Acebrophylline | 22.5 | Sustained-release layer |
| 9 | Lactose hydrate | 1.2 | |
| 10 | carnauba wax | 15.0 | |
| 11 | Eudragit L100-55 | 16.7 | |
| 12 | Magnesium stearate | 1.3 | |
| | **Total (plain tablet)** | **100.0** | |
| 13 | Opadry | 5.0 | |
| | **Total (coated agent)** | **105.0** | |

### Test Example 1: Comparative test of dissolution rate between present invention and comparative example

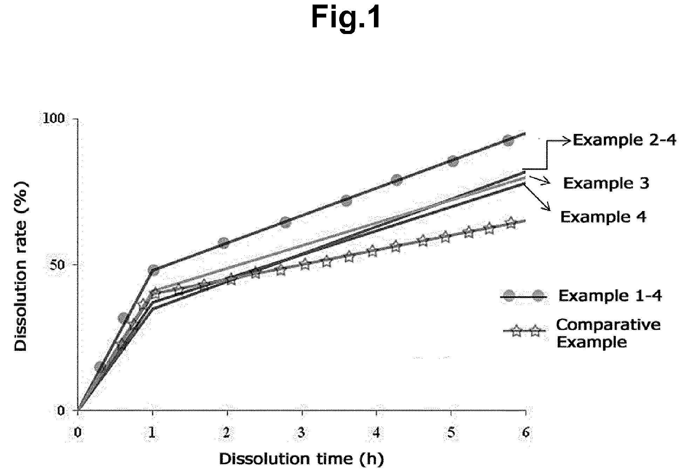

With respect to preparations of example 1-4 and example 2-4 and comparative example 1, a dissolution test was initiated in 500 mL of a dissolution medium (first fluid in Korean pharmacopoeia disintegration test) of pH 1.2 under 37±0.5□ and 50 rpm using the method 2 (paddle method) of Korean pharmacopoeia. At 2 hours after the initiation of the dissolution test, 400 mL of a test solution 2 (second fluid, 0.235 M anhydrous Na₂HPO₄ solution) was added to the dissolution liquid of pH 1.2 to make a liquid of pH 6.8, and then the dissolution test was continuously carried out. The dissolution rate was analyzed by taking the dissolution liquid at the prescribed dissolution time and checking the concentration of acebrophylline through HPLC. The dissolution rate test results over the time are shown in FIG. 1.

### HPLC analysis conditions

- Mobile phase: Acetonitrile : 0.1 % ammonium carbonate solution = 570 : 430
- Column: C18, 5µm 4.0 mm x 125 mm or similar column
- Flow rate: 1.0 mL/min
- Analysis wavelength: UV detector (244 nm)

As a result of the test, as shown in FIG. 1, the initial dissolution rates (dissolution rates at 1 hour after dissolution) of example 2-4, example 3, and example 4 (dissolution rates of 35%, 41%, and 37%, respectively), as preparations of the present invention, were similar to that of comparative example 1 (dissolution rate of about 34%) having the immediate-release layer, and with the passage of time, the preparations of the above examples showed more excellent dissolution than comparative example 1 including the sustained-release layer. The dissolution rates at 6 hours after dissolution of comparative example 1, example 2-4, example 3, and example 4 were 65%, 82%, 80%, and 78%, respectively.

Particularly, the initial dissolution rate (dissolution rate of about 47%) of the preparation of example 1-4 of the present invention was more excellent than that of comparative example 1, and with the passage of time, the preparation of example 1-4 of the present invention showed more excellent dissolution compared with comparative example 1. The dissolution rate at 6 hours after dissolution of example 1-4 was about 92%.

As such, the pharmaceutical composition of the present invention had an excellent initial dissolution rate compared with the immediate-release layer of the comparative example, thereby exhibiting fast drug efficacy, and, even with the passage of time, the pharmaceutical composition of the present invention had an excellent dissolution rate compared with the sustained-release layer of the comparative example, thereby allowing once daily administration, and thus can improve the convenience of administration and expect the improved therapeutic effect.

### Test Example 2: In vivo human PK comparision (between Sulfolase capsule and comparative example 1)

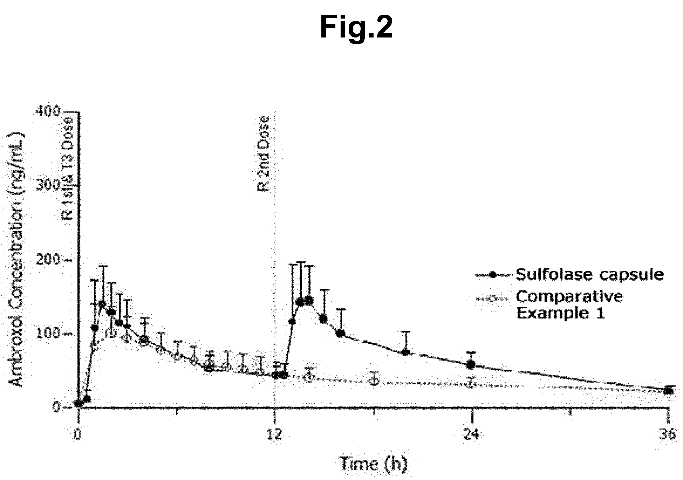

The blood ambroxol concentration when 100 mg of Surfolase® capsule (Hyundai Pharm) on the market was administered to healthy adult males (n=24) twice at 12-hour intervals was compared with the blood ambroxol concentration when the preparation of comparative example 1 (200 mg) was administered once daily through the 2x2 crossover test (table 6). The results of the comparative test are shown in FIG. 2. Since acebrophylline was metabolized into ambroxol and 7-theophylline when orally administered, the blood ambroxol concentration was measured.

As the pretreatment procedure for measuring the blood ambroxol concentration, 50 mL of a plasma sample was placed in a micro-tube, and 10 mL of a sildenafil citrate solution (10 ug/mL in 50% methanol), as an internal standard material, and 100 mL of 0.05 M a NaOH solution were added thereto, followed by vortexing for 5 seconds. 2 mL of an extraction solvent (methyl tert-butyl ether) was added thereto, followed by vortexing for 2 minutes and centrifugation at 4,000 rpm for 5 minutes. The supernatant was taken, and completely concentrated and dried in a nitrogen evaporator, and then again dissolved in 300 mL of a mixture of 0.1% formic acid and 50% methanol at 1:1 (v/v), and of this mixture, 5 mL was taken and injected into LC-MS/MS.

### <LC-MS/MS analysis conditions>

- detector: MS/MS (ESI negative ion, MRM mode)
   Ambroxol: m/z 378.99 > 263.91
   Sildenafil citrate (internal standard material): m/z 475.22 > 100.19
   Desolvation temp.: 500□
   Desolvation gas flow: 600 L/hr
   Capillary voltage: 1000 V
   Collision gas: Argon
   Nebulizing gas: Nitrogen
   Column: Fortis C18 (2.1 X 5.0 mm, 1.7 mm particle size, Fortis)
   Data processor: Target Lynx 4.1
   Mobile phase: 0.1% formic acid: methanol = 30 : 70 (v/v)
   Flow rate: 0.2 mL/min
   Injection dose: 5 mL

**[Table 6]**

| Pharmacokinetic parameter | Surfolase® capsule | Comparative example 1 |
|---|---|---|
| | Mean SD | Mean SD |
| AUCₜ (ng·hr /mL) | 2281.5 ± 763.15 | 1470.7 ± 570.36 |

As a result, twice administration of Sulfolase capsule exhibited a higher blood ambroxol concentration compared with a double-layered tablet of comparative example 1 composed of an immediate-release layer and a sustained-release layer.

### Test Example 3: In vivo human PK test (Surfolase® capsule and example 1-4)

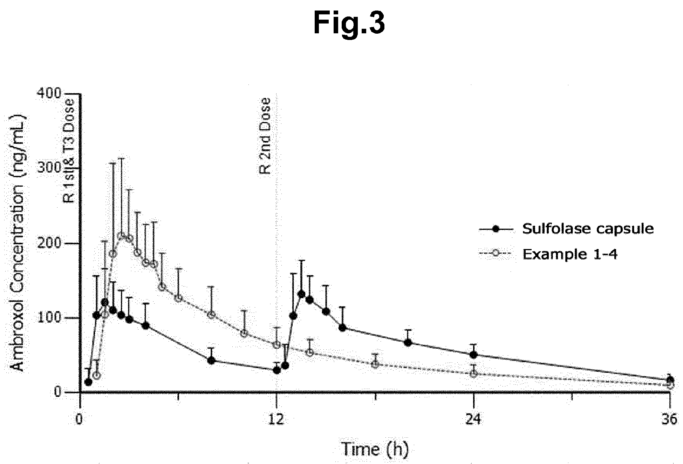

The blood ambroxol concentration when Surfolase® capsule (100 mg) on the market was administered to healthy adult males (n=24) twice at 12-hour intervals was compared with the blood ambroxol concentration when the preparation of example 1-4 (200 mg) according to the present invention was administered once daily through the 2x2 crossover test (table 7). The results of the comparative test are shown in FIG. 3.

**[Table 7]**

| Pharmacokinetic parameter | Surfolase® capsule | Example 4 |
|---|---|---|
| | Mean SD | Mean SD |
| AUCₜ (ng·hr /mL) | 2013.99 ± 522.44 | 2020.91 ± 615.46 |

As a result, only once administration of the preparation of example 1-4, according to the present invention, showed a similar blood ambroxol concentration to Surfolase® capsule on the market that needs to be administered twice daily. This validated that, though once daily administration, the convenience of administration was increased, and further the therapeutic effect was significantly improved.

In addition, the preparation of example 1-4 reached a blood concentration of about 215 ng/mL at 3 hours after administration, thereby exhibiting initial immediate-release characteristics, and then exhibited sustained-release characteristics, and these results almost coincided with the results of the dissolution rate test of test example 1.

### Test Example 4: In vivo human PK test (Sulfolase® capsule and example 2-4)

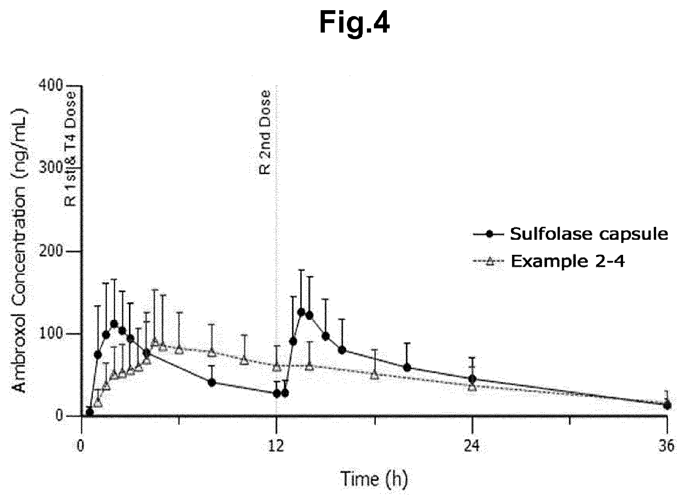

The blood ambroxol concentration when Surfolase® capsule (100 mg) on the market was administered to healthy adult males (n=24) twice at 12-hour intervals was compared with the blood ambroxol concentration when the preparation of example 2-4 (200 mg) according to the present invention was administered once daily, through the 2x2 crossover test (table 8). The results of the comparative test are shown in FIG. 4.

**[Table 8]**

| Pharmacokinetic parameter | Surfolase® capsule | Example 2-4 |
|---|---|---|
| | Mean SD | Mean SD |
| AUCₜ (ng·hr /mL) | 1830.38 ± 846.57 | 1677.84 ± 702.43 |

As a result, only once administration of the preparation of example 2-4, according to the present invention, showed a similar blood ambroxol concentration to Sulfolase capsule on the market that needs to be administered twice daily. This validated that, though once daily administration, the convenience of administration was increased, and further the therapeutic effect was significantly improved.

In addition, the preparation of example 2-4 reached a blood concentration of about 95 ng/mL at 3 hours after administration, thereby exhibiting initial immediate-release characteristics, and then exhibited sustained-release characteristics, and these results almost coincided with the results of the dissolution rate test of test example 1.

## Claims

1. A sustained-release pharmaceutical composition containing acebrophylline and a hydrophobic sustained-release agent.

2. The composition of claim 1, wherein the composition has an acebrophylline dissolution rate of 20-52 wt% at 1 hour after dissolution, and an acebrophylline dissolution rate of 75-100 wt% at 6 hours after dissolution.

3. The pharmaceutical composition of claim 1, wherein the composition is administered once daily.

4. The composition of claim 1, wherein the hydrophobic sustained-release agent is at least one selected from the group consisting of acetyltributyl citrate, carnauba wax, cellulose acetate, glycerin monostearate, glyceryl monooleate, glyceryl palmitostearate, hydrogenated castor oil, polyoxyglycerides, stearic acid, tributyl citrate, white wax, yellow wax, zein, cellulose acetate phthalate, acetyl alcohol, acetyl esters wax, dibutyl sebacate, ethylcellulose, glyceryl behenate, hydrogenated vegetable oil type I, isopropyl palmitate, polymethacrylates, shellac, stearyl alcohol, and a mixture thereof.

5. The composition of claim 1, wherein the composition is provided in a solid oral dosage form.

6. The composition of claim 1, wherein the composition is used for the prevention or treatment of at least one selected from the group consisting of acute respiratory disease, chronic respiratory disease, acute bronchitis, chronic bronchitis, bronchial asthma, sinusitis, and rhinitis sicca.

7. The composition of claim 1, wherein the composition is provided in a single matrix type tablet form.

8. The composition of claim 1, wherein the composition has both immediate-release characteristics and sustained-release characteristics.

9. The composition of claim 8, wherein the composition shows the maximal blood ambroxol concentration at 2 to 4.5 hours after once daily oral administration to the human body to exhibit immediate-release characteristics, and then exhibits sustained-release characteristics.

10. The composition of claim 1, wherein the composition has a maximal blood ambroxol concentration of 90-250 ng/mL when 200 mg of acebrophylline is orally administered once daily to the human body.

11. The composition of claim 1, wherein the composition has an AUC_{(0-36 hr)} value of ambroxol in the blood, of 1,600-2,700 ng·hr/mL, when 200 mg of acebrophylline is orally administered once daily to the human body.

12. A method for imparting initial immediate-release characteristics and late sustained-release characteristics to a pharmaceutical composition containing acebrophylline, the method including a step of adding a hydrophobic sustained-release agent to the pharmaceutical composition containing acebrophylline, followed by mixing.

13. The method of claim 12, wherein the pharmaceutical composition mixed with the hydrophobic sustained-release agent has an acebrophylline dissolution rate of 20-52 wt% at 1 hour after dissolution, and an acebrophylline dissolution rate of 75-100 wt% at 6 hours after dissolution.

14. The method of claim 12, wherein the hydrophobic sustained-release agent is at least one selected from the group consisting of acetyltributyl citrate, carnauba wax, cellulose acetate, glycerin monostearate, glyceryl monooleate, glyceryl palmitostearate, hydrogenated castor oil, polyoxyglycerides, stearic acid, tributyl citrate, white wax, yellow wax, zein, cellulose acetate phthalate, acetyl alcohol, acetyl esters wax, dibutyl sebacate, ethylcellulose, glyceryl behenate, hydrogenated vegetable oil type I, isopropyl palmitate, polymethacrylates, shellac, stearyl alcohol, and a mixture thereof.

15. The method of claim 12, wherein the pharmaceutical composition mixed with the hydrophobic sustained-release agent is provided in a solid oral dosage form.

16. The method of claim 12, wherein the pharmaceutical composition mixed with the hydrophobic sustained-release agent is provided in a single matrix type tablet form.

17. The method of claim 12, wherein the pharmaceutical composition mixed with the hydrophobic sustained-release agent shows the maximal blood ambroxol concentration at 2 to 4.5 hours after once daily oral administration to the human body to exhibit immediate-release characteristics, and then exhibits sustained-release characteristics.

18. The method of claim 12, wherein the pharmaceutical composition mixed with the hydrophobic sustained-release agent has a maximal blood ambroxol concentration of 90-250 ng/mL when 200 mg of acebrophylline is orally administered once daily to the human body.

19. The method of claim 12, wherein the pharmaceutical composition mixed with the hydrophobic sustained-release agent has an AUC_{(0-36 hr)} value of ambroxol in the blood, of 1,600-2,700 ng·hr/mL, when 200 mg of acebrophylline is orally administered once daily to the human body.
